(19) Europäisches Patentamt

European Patent Office

Office européen des brevets

(11) **EP 1 390 082 B1**

(12) **EUROPEAN PATENT SPECIFICATION**

(45) Date of publication and mention
of the grant of the patent:
**26.10.2005 Bulletin 2005/43**

(51) Int Cl.$^7$: **A61L 2/16**, G01N 31/00,
C12Q 1/22, C12M 1/00

(21) Application number: **01961547.5**

(22) Date of filing: **30.08.2001**

(86) International application number:
**PCT/SE2001/001837**

(87) International publication number:
**WO 2002/017975 (07.03.2002 Gazette 2002/10)**

(54) **METHOD FOR DETECTING OZONE CONSUMING AGENTS**

VERFAHREN ZUM AUFFINDEN VON OZONVERBRAUCHENDEN VERBINDUNGEN

PROCEDE DE DETECTION D'AGENTS CONSOMMATEURS D'OZONE

(84) Designated Contracting States:
**AT BE CH CY DE DK ES FI FR GB GR IE IT LI LU
MC NL PT SE TR**

(30) Priority: **30.08.2000 SE 0003054
02.02.2001 WOPCT/SE01/00201**

(43) Date of publication of application:
**25.02.2004 Bulletin 2004/09**

(73) Proprietor: **SC Trade & Consult AB
123 44 Farsta (SE)**

(72) Inventor: **CHOWDHURY, Sudhir
123 52 Farsta (SE)**

(74) Representative: **Allee, Harriet Eva Charlotta et al
Dr Ludwig Brann Patentbyra AB,
Box 17192
104 62 Stockholm (SE)**

(56) References cited:
**EP-A1- 0 535 612**

- **PATENT ABSTRACTS OF JAPAN vol. 199, no.
612 26 December 1996 & JP 08 215 690 A
(HITACHI LTD) 27 August 1996**
- **PATENT ABSTRACTS OF JAPAN vol. 199, no.
602 29 February 1996 & JP 07 284 782 A (HITACHI
LTD) 31 October 1995**

**Description**

The present invention relates to a method of assessing the presence of ozone consuming agents as well as to a device for use in such a method.

More specifically, the present invention relates to a method of assessing a state of cleanness, in terms of the essential absence of ozone consuming agents.

BACKGROUND OF THE INVENTION

Within some industrial fields, such as the beverage, food and pharmaceutical industry, a very clean equipment, e.g. a processing equipment, is of primordial importance. In particular, microbial and other organic contaminants should be avoided on those surfaces of the equipment which come into contact with e.g. food and pharmaceutical components. Consequently, the state of cleanness of the equipment must in general be assessed on a regular basis and cleaning of the equipment must in general be regularly performed.

When the equipment to be cleaned comprises e.g. a system of pipes and vessels which are not easily dismounted, cleaning of this system is made in place, i.e. as a CIP (cleaning in place) process.

In relation to organic and microbial contamination, the present ways of assessing cleanness of an equipment of the above described type is by manual sampling of the organic and/or microbial contaminants possibly present on the interior surfaces of the equipment; transfer of the samples to a culture medium, where the possible microbes are cultured for some time; counting and possibly identifying the microbial colonies so obtained.

Also, examination of the samples by means of UV microscopy to detect organic pollutants is a method which is equally cumbersome and time consuming.

It is evident that these methods are quite unreliable: For example, sampling is of an inherent random character, and sampling and culturing are dependent on the professional skill of the person performing such work, and moreover, there may be surfaces within the equipment which are in fact not accessible to sampling. As a result, this method may on the best give only an indication that the equipment is probably clean. Consequently, in case this indication later reveals false, product may have to be discarded as contaminated.

Apart from being unreliable, the above outlined method of assessing cleanness is also quite time-consuming, since only culturing the microorganisms takes some 2-4 days, leading to a substantial delay between the moment of sampling and the moment of obtaining the sought results, i.e. indication of the state of cleanness of the equipment.

WO99/46201 discloses a method wherein a water having known ozone concentration is injected into an enclosure downstream of which the ozone concentration is measure. The difference of the concentrations is taken as measure for the amount of ozone consuming species present in the enclosure.

The objective problem to be solved by the present invention is to provide a method wherein account is also taken of the auto-decomposition of ozone in the absence of ozone consuming species, making the method more reliable.

Also within other fields of application it may be of a great importance to be able to monitor cleanness of a system in a quick and reliable way, i.e. preferably on-line and in real time. Such a system may be any enclosure susceptible of being contaminated with ozone consuming agents, such as organic pollutants or microbes. An example of such an enclosure is a compression chamber as used to simulate submarine conditions in the naval training of submarine staff. Another example is the hyperbaric chamber used within the filed of marine petroleum drilling.

Also, in a decompression chamber such as may be used in treatment of e.g. persons suffering from serious burn injuries, or persons suffering from aeroembolism, the state of cleanness, in terms of the absence of harmful microorganisms, may be of uttermost importance.

SUMMARY OF THE INVENTION

According to one aspect the present invention provides a method of assessing the presence of ozone consuming agents on the surface of an object or within an enclosed volume, by providing a fluid containing ozone, bringing the fluid into contact with the object or introducing the fluid into the enclosed volume, measuring the concentration of ozone in the fluid and evaluating the presence of the ozone consuming agents as a function of the measured ozone concentration.

According to another aspect the present invention provides a device for use in such a method, comprising at least one source of a fluid containing ozone, means for bringing the fluid into contact with the surface or introducing it into the enclosed volume, and means for measuring the ozone concentration in the fluid.

In still another aspect of the invention a method of assessing the presence of ozone consuming agents on the surface of an object or within an enclosed volume is provided wherein the fluid phase is maintained in circulation.

In a preferred aspect of the invention, the fluid is maintained in circulation and the ozone concentration is measured in at least two points, at least one point being situated so as to provide a measure of the concentration of ozone in the fluid not having been brought into contact with the object and not having been introduced into the enclosed volume, which concentration is taken as the reference concentration, the other one being situated so as to provide a measure of the ozone concentration of the fluid having been brought into contact with the object or introduced into the enclosed volume.

According to one aspect the invention provides a method of assessing the cleanness, expressed in terms of the essential absence of ozone consuming agents, of a surface or volume, and a device therefor.

The invention is further defined in the claims.

BRIEF DESCRIPTION OF THE FIGURES

Figure 1 is a schematic representation of a device of the invention for use in a method of assessing the cleanness of e.g. a processing equipment.

Figure 2 is a schematic cross-sectional view of an embodiment of a mixing chamber according to the invention.

Figure 3 is a schematic cross-sectional view of another embodiment of a mixing chamber according to the invention

Figure 4 is a schematic cross-sectional view of still another embodiment of a mixing chamber according to the invention.

Figure 5 is a bar diagram representing the measured ozone concentration of a closed loop (spotted bars) in the absence of contamination as compared to the ozone concentration measured in a reference point (bars filled with dashed lines).

Figure 6 is a bar diagram representing the measured ozone concentration of a moderately contaminated closed loop (spotted bars) as compared to the ozone concentration measured in a reference point (bars with dashed lines).

Figure 7 is a bar diagram representing the measured ozone concentration of a heavily contaminated closed loop (spotted bars) as compared to the ozone concentration measured in a reference point (bars with dashed lines).

DETAILED DESCRIPTION OF THE INVENTION

In one embodiment of the invention, a fluid phase containing ozone is brought into contact with an object susceptible of comprising ozone consuming agents on its surface, the ozone concentration of the fluid phase is measured and the presence of ozone consuming agents is assessed as a function of the measured ozone concentration in the fluid phase.

In another embodiment of the invention, a fluid phase containing ozone is introduced into an enclosed volume, susceptible of comprising ozone consuming agents, the ozone concentration of the fluid phase is measured and the presence of ozone consuming agents within the volume is assessed as a function of the measured ozone concentration in the fluid phase.

In the case of an enclosed volume, i.e. a volume of space surrounded by an enclosing surface, i.e. an enclosure, it should be understood that the ozone consuming agents may be present as well on the surface of the enclosure facing the volume, e.g. adsorbed on the surface of its inside, or at distance from the surface within the volume. An example would be an enclosure filled with a gas such as air, wherein microorganisms and/ or chemical compounds capable of reacting with ozone are susceptible of being present as well in the gas phase as e.g. adhered or adsorbed to the inner surface of the enclosure.

Consequently, it may be desired either to assess cleanness of the entire enclosed volume, i.e. of the inside of the enclosing walls, as well as of the material and/or objects contained therein, as in the case of a gas filled chamber, e.g. a compression or decompression chamber, or alternatively it may be desired to assess cleanness of only the inner walls of the enclosure as well as any other surface interior to the enclosure and which may come into contact with the ozone containing fluid, such as the surface of any object(s) located inside the enclosure.

Also, it should be understood that the enclosure may be of any type, i.e. a chamber, a vessel, a piping, conduit, a system of any of the former connected to each other, such as in a processing equipment etc.

The ozone concentration in the fluid phase is measured continuously or intermittently, and may be expressed as a ratio of the initially measured concentration or of a predetermined concentration of ozone in the fluid phase, which ratio, when expressed as a percentage, theoretically may vary from 100% to 0%. In the absence of ozone consuming agents on the object or within the enclosure, ozone in the fluid phase will remain without any substantial decomposition during at least the measurement period and thus the above mentioned ratio will remain essentially constant. In the presence of ozone consuming agents, on the contrary, ozone will react rapidly therewith and the ozone concentration will decrease rapidly.

The initial concentration of ozone in the fluid phase which is brought into contact with the object or introduced into the enclosure suitably may range from 5 ppb (parts per billion, i.e., 1 part of ozone per 1 billion parts of fluid) to 10 ppm (parts per million, i.e. 1 part of ozone per 1 million parts of fluid), more specifically from 5 ppb to 5 ppm, all parts referring to the weight.

The method of determining the ozone concentration in the fluid phase preferably should conform to the standards of IOA (International Ozone Association), such as the standards 006/89(F) Colorimetric method for the determination of traces of ozone in water (indigo-trisulphonate-method); 007/89(F) Electrochemical method for continuous measurement of residual ozone in water; 004/89(F) Colorimetric method for the determination of residual ozone in water (indigo-trisulphonate-method), 002/87(F) Ozone Concentration Measurement In A Process Gas By UV Absorption; 008/89 (F) Photometric Measurement of Low Ozone Concentrations In the Gas Phase; 001/87 (F) Iodometric method For The Determination Of Ozone In A Process Gas, available from International Ozone Association Standardisation Committee c/o CIBE, 764 Chaussée de Waterloo, B-1180 Brussels. Provided this requirement is met, any method may be used showing selectivity for ozone coupled with a high sensitivity and accuracy. When, for example, the fluid phase is gaseous, ozone concentration may be determined by UV absorption photometry. When, on the other hand the fluid phase is liquid, ozone concentration may suitably be determined by use of a polarographic membrane electrode as well as newly developed UV absorption photometer for ozone in liquid.

In one embodiment, the fluid phase containing ozone is made to circulate in contact with the object or through the enclosure, and the ozone concentration of the circulating fluid phase is measured continuously or intermittently. A volume flow rate of the circulating fluid phase of from 0.1 ml/min to 30 l/min, more specifically from 0.1 ml/min to 5 l/min, may suitably be selected.

The fluid phase containing ozone may be produced by feeding, into a suitable vessel such as a mixing chamber, ozone from a source of ozone, and fluid phase from a source of fluid and mixing the two components within the vessel.

By fluid phase is meant any suitable phase which is fluid at the temperature and pressure applied. It may be a gaseous phase of a chemically inert gas or a mixture of chemically inert gases, i.e. essentially not reacting with either the ozone or the ozone consuming agents. An example of such a gas would be $CO_2$. It also may be a liquid phase, such as an aqueous phase, e.g. water.

The fluid phase may have a temperature of from 0 °C up to under 60 °C but suitably has a temperature of less than 30 °C, more suitably less than 20 °C, preferably less than 15 °C more preferably less than 10 °C, and especially around 5 °C. Cooling means, such as a conventional cooler, may be provided to refrigerate the fluid to the selected temperature.

Ozone may be produced from oxygen in an ozone generator, as known to the person skilled in the art. Pure ozone conveniently is produced from oxygen in a solid state ozone generator, minimising the production of NOx.

The device may suitably comprise means for eliminating excess ozone. Such means may comprise e.g. a degasifier and ozone destruction units, such as those providing catalytic or thermal destruction of excess ozone.

Optionally, the excess ozone may be recycled and used e.g. in a disinfection application or for a pre-treatment purpose.

The entire process of assessing the presence of ozone consuming agents may be automated, using a suitably programmed data processing and control unit, such as a computer device.

Data from the analysis of the ozone concentration in the fluid phase, as well as other data from the method, such as temperature of the fluid phase, flow rate etc may be stored by the computer device, which may also be used to control various parameters of the method, such as the initial ozone concentration, the water quality, the volume flow rate, the temperature of the fluid phase etc. Parameters and data such as ozone concentration may be visualized on a display screen and/or plotted by a curve printer connected to the computer device.

It should be noted that the surface, on which the ozone consuming agents are susceptible of being present or enclosing the volume within which the ozone consuming agents are susceptible of being present, in itself should not in any essential way affect the concentration of ozone in the fluid phase, e.g. should be substantially chemically inert towards ozone and show no substantial adsorptive properties towards ozone. Thus, the surface material should not in itself be degradable by contact with ozone in the fluid at the concentrations employed. Evidently, the same applies to any material of the device for carrying out the method of the invention susceptible of coming into contact with the ozone containing fluid. Thus, for parts susceptible of coming into contact with the ozone containing fluid, e.g. rubber would not be a suitable material, whereas e.g. silicone, Teflon, stainless steel, would fulfil the above requirements.

The exact kind of the object susceptible of carrying on its surface ozone consuming agents, as well as the exact kind of enclosure susceptible of comprising within it ozone consuming agents, are not critical to the invention, as long as the material which is to be brought into contact with the ozone containing fluid fulfils the above requirements.

As an example, the object could be a single item, such as an appliance or tool, or could be parts of an equipment, such as e.g. a medical or processing equipment which is contaminated by organic materials and microorganisms.

As used in the present application, the term processing equipment should be read in a wide sense and understood to comprise any equipment intended for containing, transporting, processing etc liquid, solid and/or gaseous material, such as pipings, vessels of different kinds, as well as blades, knives, nozzles etc. The processing equipment could be of the kind used in food, pharmaceutical, beverage industry, but could also be found in any field where it is desirable to be able to control cleanness.

The enclosure may be e.g. a decompression chamber, compression chamber or hyperbaric chamber, or a chamber or vessel forming part of any equipment, such as a medical or processing equipment.

The method of the present invention may advantageously be applied to assess the cleanness of any object, in terms of the absence of ozone consuming agents. In one embodiment, the method is applied to assess the cleanness of an enclosed volume.

In one embodiment where the fluid phase is a gas phase, the method may be performed by introducing, into an enclosed gas-containing volume, an ozone containing gas at a slight overpressure. After a certain holding time, the ozone concentration of the gas phase of the enclosed is measured, e.g. by introducing a sample thereof into an ozone measuring apparatus, and the measured ozone concentration is compared to a suitable reference. Such reference may be e.g. the ozone concentration of the enclosed volume at an earlier point of time, such as directly after completing the introduction. It may also be the ozone concentration of the gas phase as introduced into the enclosed volume, corrected for the dilution within the enclosed volume and possibly for the ozone autodestruction and the for reaction of ozone with the surface material of the inner walls of the enclosure. Of course, as already stated herein, this surface material preferably should be essentially inert to the ozone gas.

In one further embodiment, a flow of the ozone containing gas phase is continuously introduced into the enclosed volume, a gaseous flow is continuously withdrawn therefrom, preferably at essentially the same rate, and the ozone concentration of the withdrawn gas is measured. After a certain purging time the measured ozone concentration in the withdrawn gaseous flow will give an indication of the purity of the system, taking e.g. the inlet side ozone concentration as a reference value.

It should be noted that in a closed gaseous system free from any ozone consuming agents, such as clean air free from e.g. microorganisms and other pollutants, ozone at low concentration may remain essentially stable for several hours or even days.

As an example, into an enclosure having a volume of 1 $m^3$ a gaseous flow containing ozone at a known concentration, such as from 0.1 to 10 ppm is introduced at a flow rate of e.g. 1000 ml/min and at a low overpressure, such as a positive pressure of e.g. 0.1-0.5 bars. After at least 10 minutes of purging the enclosure with the ozone containing gas, follows a holding time of e.g. 5 minutes and the ozone concentration may then be measured to provide an indication of the state of cleanness of the enclosure.

In one embodiment, the method is applied to assess the cleanness of surfaces or volumes within an enclosed volume which is a processing equipment.

In an advantageous embodiment, the fluid phase is an aqueous phase, preferably water, such as municipal tap water, distilled water or deionized water.

In an embodiment of the invention, water containing ozone is introduced into a processing equipment, the cleanness of which is to be assessed, and measurement is made of the ozone concentration in at least one point within the equipment.

The ozone concentration in the water is measured continuously or intermittently, and may be expressed e.g. as a ratio of the initially measured concentration, which ratio theoretically may vary from 100 % to 0%. In the absence of ozone consuming contamination in the equipment, such as organic matter and microorganisms, such as bacteria and/or spores, ozone in the water will remain without any substantial decomposition during at least the measurement period and thus the above mentioned ratio will remain essentially constant. In the presence of any ozone consuming contamination, on the contrary, ozone will react rapidly therewith and the ozone concentration will decrease rapidly.

The initial concentration of the ozone in the water to be injected into the equipment usefully may be from 5 ppb (parts per billion, i.e., 1 part of ozone per 1 billion parts of water) to 10 ppm (parts per million, i.e., 1 part of ozone per 1 million parts of water), more specifically from 5 ppb to 5 ppm, all parts referring to the weight.

In the absence of organic contamination and/or microorganisms within the equipment, ozone concentration may be substantially stable over as long as 60 minutes or more. This stability of ozone in the water is a very advantageous feature obtained by a special way of mixing ozone and water according to the invention, as will be described herein below.

In one embodiment, water containing ozone is injected as a continuous flow through the equipment, and the ozone concentration is measured continuously or intermittently in at least one point within or in connection to the equipment. A flow rate of water containing ozone of from 0.1 ml/min to 30 l/min, more specifically from 0.1 ml/min to 5 l/min, may suitably be selected.

Water containing ozone may be produced by feeding, into a suitable vessel such as a mixing chamber, ozone from a source of ozone, and water from a source of water and mixing the two components within the vessel. The mixing time required to obtain a homogeneous solution depends i.a. on the volume of water in the tank. For a volume of water of 5 litres to 10 000 litres, a mixing time of from approximately 2 minutes to 20 minutes will be sufficient. An advantageous way of obtaining a homogeneous solution is to circulate water at a low flow rate and to inject ozone into the circulating water.

The water used may be e.g. municipal tap water, distilled water or deionized water. It may have a temperature of from above 0 °C up to approximately 60 °C, but suitably has a temperature of less than 30 °C, more suitably less than 20 °C, preferably less than 15 °C more preferably less than 10 °C, and especially around 5 °C.

A specification of broad and preferred ranges of characteristics of water for use in the system is given in Table 1:

Table 1:

| Characteristics of water for use as fluid phase in a method of the invention | | |
|---|---|---|
| Characteristic | Broad range | Preferred range |
| pH | 3-8 | 4-7 |
| Conductivity (mS/cm) | <0.2 | <0.02 |
| Temperature (°C) | 3-20 | 3-7 |
| COD (mg/l) | 1-2 | <0.02 |
| Colour (mg/l Pt) | <5 | 0 |
| Content of small particles | Not more than 15 particles smaller than 1 μm /ml | Not more than 15 particles smaller than 2 μm/ml |
| Note: COD stands for Chemical Oxygen Demand | | |

The mixing vessel may suitably be connected to means for separation and destruction of ozone released from the liquid phase into the overhead gas phase. Such means may comprise a degasifier and a destruction unit.

The obtained mixture of water and ozone is introduced into the processing equipment, and optionally circulated through this equipment at a selected flow rate. The introduction may be accomplished by use of e.g. a pumping means. A suitable pumping means would be e.g. a regenerative turbine pump.

The concentration of ozone is measured in at least two points within the processing equipment or in direct connection therewith, whereby at least one point should be located within the processing equipment or beyond it, i.e. downstream of it, and the other one, being taken as a reference, should be situated upstream of the processing equipment or in a reference point parallel to it. For example, measurement may be made of the ozone concentration in the mixing vessel, anywhere between the mixing vessel and the processing equipment, within the processing equipment and/or in a point beyond the processing equipment, i.e. situated beyond the outlet of the equipment.

The ozone concentration in the water in any of the above mentioned points may suitably be determined by polarographic analysis or by dissolved UV-analyser.

It should be noted that the materials of all those parts that may come into contact with the ozonized water, i. e. parts of the processing equipment as well as of the device according to the invention, should not in any essential way affect the concentration of ozone in the water, e.g. should be substantially chemically inert towards ozone and show no substantial adsorptive properties towards ozone. The material in contact with the ozonized water of course should not in itself be degraded by contact with the ozone in the water. Suitable materials would be high grade acid-proof stainless steel, titanium, PVC (polyvinylchloride), PVDF (polyvinylidene fluoride), Teflon or silicone material.

The entire process of assessing the cleanness of a processing equipment may be automated, using a suitably programmed data processing and control unit, such as a computer device, and communication for evaluation of data may be performed via internet.

Data from the analysis of the ozone concentration as well as other data from the method, such as pH and temperature of the water, flow rate, water quality etc. may be stored by memory means of the computer device. Laboratory made calibration curves should suitably also be stored from time to time to give the operator an opportunity to validate the function and repeatability of the measuring method. The computer device may be used to control parameters of the method, such as pH, temperature and initial ozone concentration of the fluid phase. Ozone concentration may be visualized on a display screen and/or plotted by a curve printer connected to the computer device.

The control unit may also perform a direct control of the cleaning process, such that the cleaning process will be reinitiated/initiated by the control unit as a function of measured ozone concentration.

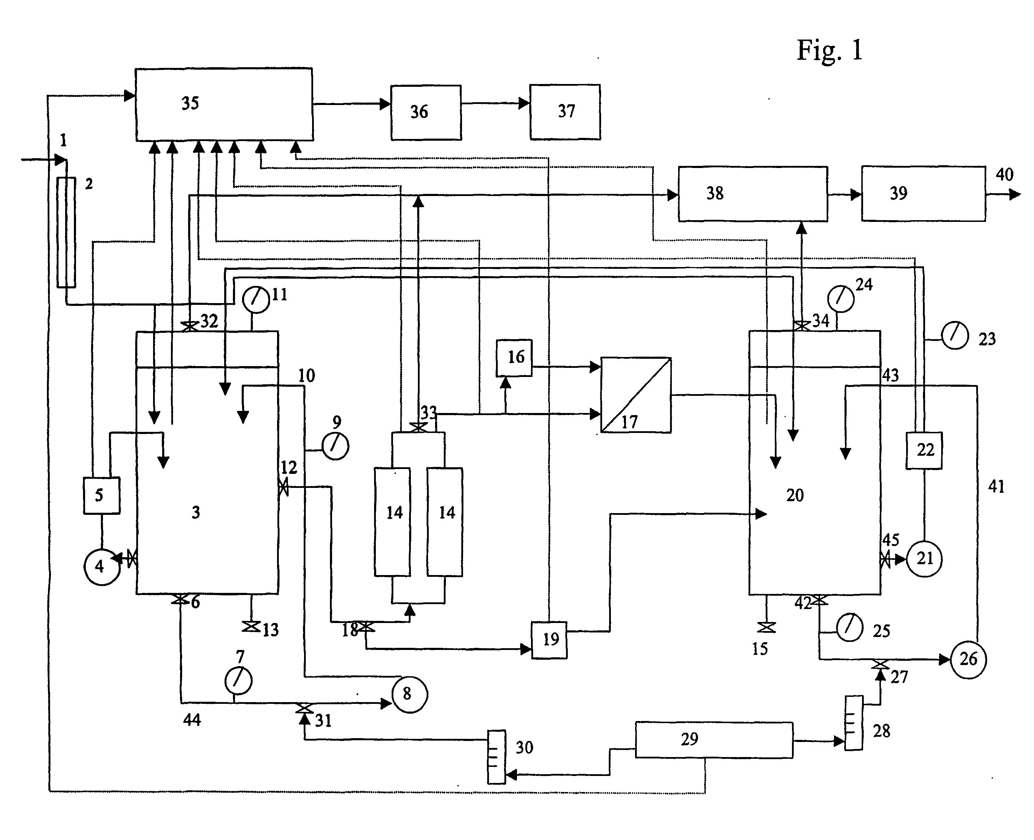

A preferred embodiment of the invention will now be described, with reference to Fig.1 of the appended drawing, schematically representing a device for use in a method of the invention.

The device represented in Fig.1 basically comprises an inlet 1 for water circulating in fluid conduits of the device, a water cooler 2, an ozone generator 29, two mixing tanks 3, 20, wherein water and ozone are mixed together, both provided with ozone injection loops 41, 44 wherein ozone is injected into a flow of water; a number of ozone measuring units 5,16, 19, 22 for measuring the aqueous ozone concentration at different points within the device; a number of flow meters 28, 30, valves 6, 12, 13, 15, 18, 27, 31, 32, 34, 42, 45 and pressure gauges 7, 9, 11, 23, 24, 25 for measuring and regulating flow and pressure within the device; a unit for elimination of excess ozone comprising valves 32 and 34, degasifier 38 and an ozone destructor 39 with an outlet 40; a particle filter 17; and a data processing and control unit comprising a microprocessor 35, a computer 36 and a printer 37.

Referring to Fig.1, operation of the device will now be described. Water of known quality, i.e. ordinary municipal tap water, distilled water or deionized water, is fed via inlet 1, into cooler 2, where it is refrigerated to a temperature of $5 \pm 1\,°C$, and then passed into mixing tank 3.

When a pre-selected volume of water has been fed into mixing tank 3, pump 8 starts pumping water through ozone injection loop 44 and pump 4 starts pumping water through a measuring loop comprising an ozone measuring means 5, e.g. a temperature compensating polarographic membrane electrode, providing continuous measurement of the ozone concentration of the water in mixing tank 3.

Mixing tanks 3 and 20 are connected to each other so that water may flow from mixing tank 3, into mixing tank 20 and back again into mixing tank 3. To this end, valves 12, 45 are provided on the mixing tanks 3 and 20 respectively, and circulation of water is obtained by means of pump 21 on the outlet side of mixing tank 20.

As stated above, both mixing tanks 3, 20 are provided with an ozone injection loop 41, 44, wherein ozone is injected into water. To inject ozone into the water, a flow of water is withdrawn through the outlet valve 6, 42, situated on the bottom of the tank 3, 20, and circulated through the ozone injection loop 41, 44 by means of a pump 8, 26. By means of outlet valve 6, 42, located to permit a flow generated by the gravity, an essentially zero positive pressure is obtained on the inlet side of pump 8, 26; at the outlet side of pump 8, 26 the pressure is 1.6-3 bars. Pressure gauges 7, 9, 25, 44 are provided on the ozone injection loop to monitor pressure within the loop. Ozone is generated from oxygen gas by ozone generator 29 and a flow of ozone, regulated by flow meter 30, 28 is injected into the flow of water through valve 31,27 with a positive pressure of 0.01-2 bars, e.g. 0.1-0.5 bars. The water flow, now containing ozone, is returned to mixing tank 3, 20 entering through inlet 10, 43 located below the surface of the pre-selected volume of water in the tank 3, 20.

In the mixing tank 3, 20 the pressure, which should not exceed around 0.6 bar, is monitored by means of pressure gauge 11, 24. In case of a pressure rise above said limit, nonreturn valves 32, 34 will open to restore pressure within mixing tank 3, 20 to a permissible value.

On the bottom of mixing tanks 3, 20, valves 13, 15 are provided to permit an easy emptying of liquid.

The specific feature of introducing ozone gas at a low positive pressure, as specified herein above, into water as characterized in Table 1, having essentially zero positive pressure, will provide an aqueous solution of ozone of a very advantageous stability over time. As an example, aqueous ozone concentration has been followed over different periods of time, up to more than 60 minutes, at different initial concentration levels. The results are compiled in Table 2.

Table 2.

| Variation of aqueous ozone concentration over time | |
| --- | --- |
| Initial ozone concentration in municipal tap water (ppm) | Ozone concentration (ppm) in municipal tap water after time (t min) |
| 9 | 6.5 (60min) |
| 5 | 3.8 (60 min) |
| 0.5 | 0.4 (20 min) |
| 0.4 | 0.3 (20 min) |

Of course, stability of the ozone is dependent on the quality of the water employed. In an experimental set-up, the ozone concentration has been found to be essentially stable for more than 30 minutes (>30 min) in municipal tap water; for more than 60 minutes (>60 min) in deionized water; and for more than 100 minutes (>100 min) in distilled water.

On the other hand, in the presence of organic contaminants, ozone reacts very rapidly therewith, and the concentration of ozone in the water consequently decreases extremely rapidly.

The aqueous solution of ozone obtained according to the embodiment described above as a further advantage provides a very efficient and potent disinfecting agent. By bringing an object, contaminated by microorganisms, such as bacteria, into contact with the aqueous ozone solution, a thorough disinfection thereof consequently will be obtained.

The amount of ozone injected through valve 31 is a function of the desired initial ozone concentration in the water to be injected into the equipment, the cleanness of which is to be assessed, and the volume of water in mixing tank 3. In an initial stage of starting up the system of the invention, ozone concentration will gradually build up in the water within mixing tank 3. After this initial build-up stage, a stable pre-selected value of ozone concentration is reached, as measured in measuring loop 5. When this stable pre-selected value is reached, the ozone generator 29 is switched off and valve 31 is closed. To maintain a stable ozone concentration reference value, water in tank 3 is kept in circulation.

The device comprises ozone measuring means 5, 16, 19 and 22, respectively situated on the conduit of the measuring loop of mixing tank 3, downstream of the equipment 14, in between three-port valve 18 and mixing tank 20, and downstream of pump 21. In this way, ozone concentration may be monitored in parallel at several different locations within the system: ozone measuring means 5 gives the ozone concentration of the water within the mixing tank 3, ozone measuring means 16 gives the ozone concentration of the water after passage through the equipment 14, ozone measuring means 19 gives the ozone concentration of the water flowing from mixing tank 3 to mixing tank 20, and ozone measuring means 22 gives the ozone concentration of the water flowing from mixing tank 20 back to mixing tank 3.

As mentioned herein above, the ozone concentration of the water in mixing tank 3 is continuously measured by ozone measuring means 5, and when a desired pre-selected value has been stably reached, the ozone generator 29 is automatically switched off. However, if the ozone concentration within mixing tank 3 decreases to under a pre-selected value, as measured by ozone measuring means 5, ozone generator 29 automatically switches on again and injection of ozone is resumed so as to re-establish the desired pre-selected ozone concentration in mixing tank 3.

In order to assess the cleanness of equipment 14, the equipment 14 is connected to the device of the invention by means of three-port valve 18. Downstream of the equipment 14 to be tested, a particle filter 17 is provided on the fluid conduit, to eliminate any contaminating particles which the flow of water might wash off from the equipment 14.

To perform the assessment, valve 12 is switched open and a flow of ozone-containing water is withdrawn through the action of pump 21. The water flow passes through three-port valve 18, where it is divided into two flows: one passing into the equipment 14 and the other passing directly into ozone measuring means 19 and subsequently into mixing tank 20. Hereby, the pressure is the same in the whole fluid system, comprising as well mixing tank 3, 20 as equipment 14, i.e. around 0.6 bar.

On the other hand, in an advantageous embodiment, auxiliary mixing tank 20 is used also as a supply of ozone-containing water during the build-up of the ozone concentration in mixing tank 3. In this embodiment, water containing ozone, previously generated by ozone generator 29 and injected through valve 27 as previously described, is pumped from tank 20 into mixing tank 3 by means of pump 21 during the start-up period of the device. In this way, the start-up period of the system may be shortened, and the cleanness evaluation obtained even quicker. Tank 20 also may be used as an auxiliary supply of ozone-containing water e.g. in any situation of disruption of water supply from the external source.

In another embodiment, the ozone containing gas not having dissolved in water within mixing tanks 3 and 20, instead of being passed to ozone destruction unit 39, is recycled to be used e.g. in a disinfection application.

In a simplified device, auxiliary mixing tank 20 and the parts of the system associated therewith, such as flow meter 28, valve 27 etc. may be omitted entirely, particle filter 17 at its downstream side thereby being connected directly to mixing tank 3.

In one embodiment, water is made to circulate in the system. Ozone is introduced into the circulating water whereby measurement of the ozone concentration in 5 and 16 is initiated. At the ozone introduction, the ozone concentration will build up from a low value to a higher one both in 5 and 16, at a different rate depending on the state of cleanness of equipment 14. When a satisfying ratio is obtained between the values measured by ozone measuring means 5 and 16, respectively, the ozone generator may be switched off and the system rinsed by water.

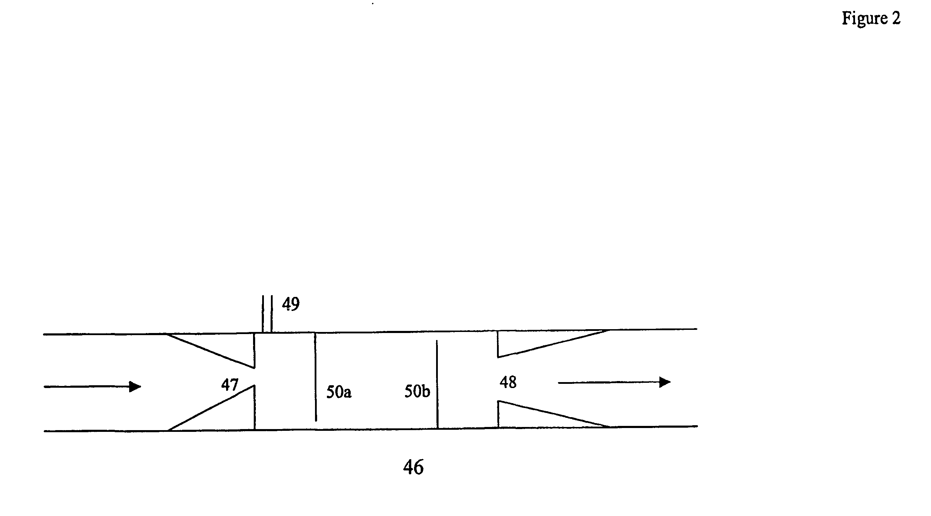

In one embodiment of the invention valves 31 and/or 27 are replaced by an ozone mixing chamber(s) 46, illustrated in Figs. 2-4, the mixing tanks 3 and/or 20 then rather performing the function of holding tanks. Introduction of the ozone containing gas into the flow of water within mixing chamber 46 is obtained by a venturi effect. Within the mixing chamber 46 the flow of water and introduced ozone containing gas is a turbulent flow, due to the special geometry of the mixing chamber 46. In this case, mixing of ozone and water obviously occurs

Introduction of ozone into the water flow is facilitated by generating a pressure drop in the flow at the point of introduction of the gas, i.e. by a venturi effect. For example, by providing a device having an inlet opening for the water flow and an outlet opening for the liquid flow, and having an inlet for the gaseous flow in a sidewall, and wherein the liquid inlet opening is substantially narrower than the liquid outlet opening, e.g. having a diameter which is 1.5-5 times smaller, more preferably 2-3 times smaller, introduction of the flow of gas into the liquid flow is through the gas inlet is facilitated. In general, a conventional venturi tube-shaped device, such as is described e.g. in McGraw-Hill Encyclopedia of Science & Technology, volume 14, pp. 346 (1977) will be appropriate. The gas flow inlet of the gas introduction device preferably is preceded by a nonreturn valve or functionally equivalent means, to prevent backflow, e.g. into the ozone generator.

In a preferred embodiment, the venturi tube-shaped device is the mixing chamber of the apparatus of the invention. The mixing chamber provides a thorough mixing of ozone and water. This is accomplished by the mixing chamber having an inner geometry which provides a turbulent flow therein.

In one embodiment of a mixing chamber having such an inner geometry, the mixing chamber is equipped with at least two baffles, located at a distance from each other in the flow direction, and extending inwards in an essentially opposite direction to each other from the inner walls and in an essentially perpendicular direction to the water flow. Each baffle should extend over more than 50% or more than 75%, preferably more than 90% or about 95% of the diameter of a section of the mixing chamber essentially perpendicular to the water flow. For example, in a mixing chamber having an inner diameter of 20 mm, a passage of 1-2 mm may be left open by the inwardly extending baffle.

In another embodiment of a mixing chamber according to the invention, the mixing chamber comprises a restriction located at a distance from the inlet of the mixing chamber as well as the outlet of the mixing chamber, and an obstruction located at a small distance downstream of this restriction. By forcing thus the flow of water and gas through the restriction and providing an obstacle on which the flow impinges, turbulence is enhanced. The restriction may be provided by two baffles, located directly opposite to each other and extending inwards from the side walls over less than 50% of the diameter of the mixing chamber so as to leave a narrow slot open for the flow. Alternatively, it may be provided by one plate, integral with the walls of the mixing chamber and having a suitably shaped aperture for the flow of water.

In one embodiment of a mixing chamber having an inner geometry suitable to provide a turbulent flow, the inner geometry is obtained by use of a filler material, providing a suitable obstacle to the flow, without creating an excessive pressure build-up, e.g. beads of suitable size.

The dimension of the mixing chamber may be selected according to the desired liquid flow. Generally, however the mixing chamber will have a length of less than 200 mm, e.g. from 200 to 100 mm. The mixing chamber has a generally narrow width, e.g. from 50 to 15 mm, which also serves to enhance the mixing of the ozone and the water.

The term "inner", as used herein in relation to the geometry of the mixing chamber, should be understood to refer to the geometry inside the chamber, i.e. the shape of those parts within the chamber which face the lumen of the same, and which are susceptible of coming into contact with the flowing water. They may be integral or not with the chamber. As an example of integral parts providing a turbulent flow, baffles extending from side walls may be mentioned. As an example of non-integral parts, a filling material, such as in the form of beads, may be mentioned, for example beads having a diameter of 0.5-4.00 mm.

The material of the walls of mixing chamber 46 may be any material which is suitable for the intended use, e.g. withstands the pressure of the flow of water, and may be e.g. Teflon® or stainless steel. However, in case the walls of mixing chamber are made from a material which is destructive to ozone, the inside of these walls should be coated with an appropriate material. Also, any filler material introduced into the mixing chamber as a means of obtaining a turbulent flow should be essentially inert towards ozone. An example of a suitable material for the fillings would be glass, Teflon® or stainless steel.

In one embodiment of the invention, surfaces inside the mixing chamber which are susceptible of contacting the water flow will be coated with a material which is both highly inert to ozone and resistant to fouling by microorganisms. Such a material may be selected from e.g. silicone, Teflon® and titanium dioxide. More preferably, this coating will cover the entire surface area within the mixing chamber susceptible of coming into contact with the flow of water.

In another embodiment of the invention, surfaces inside the mixing chamber which are susceptible of contacting the water flow will be coated with a material which has a catalytic activity such as platinum on activated carbon or on alumina, Pt/C or $Pt/Al_2O_3$.

Fig. 2 represents one embodiment of mixing chamber 46, having an inlet opening 47 and an outlet opening 48 for the water flow as well as an inlet opening 49 for the gaseous ozone containing flow in a side wall in close vicinity to the inlet opening for the water flow. The inlet opening 47 is narrower than the outlet opening 48. At a distance from the inlet side a first baffle 50a is located, followed by a second baffle 50b further downstream. The baffles 50a, 50b, both rigid and solid (integral) with the inner wall, extend from a side wall in a direction substantially perpendicular to the flow of water and in substantially opposite direction to each other.

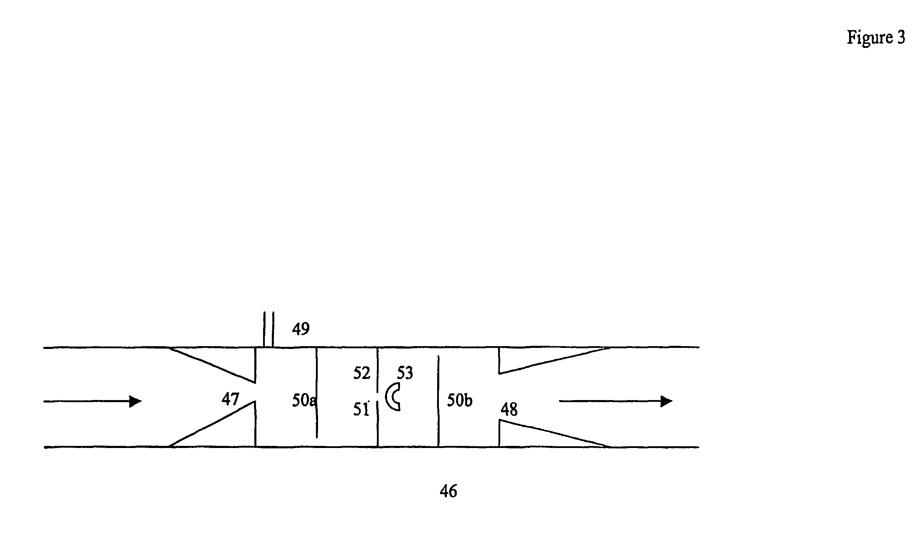

Fig. 3 represents a further embodiment of mixing chamber 46, having additionally a restriction for the flow of water downstream of baffle 50a. This restriction is provided by means of a rigid plate 51, solid with the inner walls of mixing chamber 46, having a small opening 52 for the flow of water. A curved baffle 53, located downstream of plate 51 and facing the opening 52, provides an obstacle to the flow emerging through opening 52. The curved baffle 53, with its convex side towards the opening, will provide an impact surface further enhancing turbulence in the water flow.

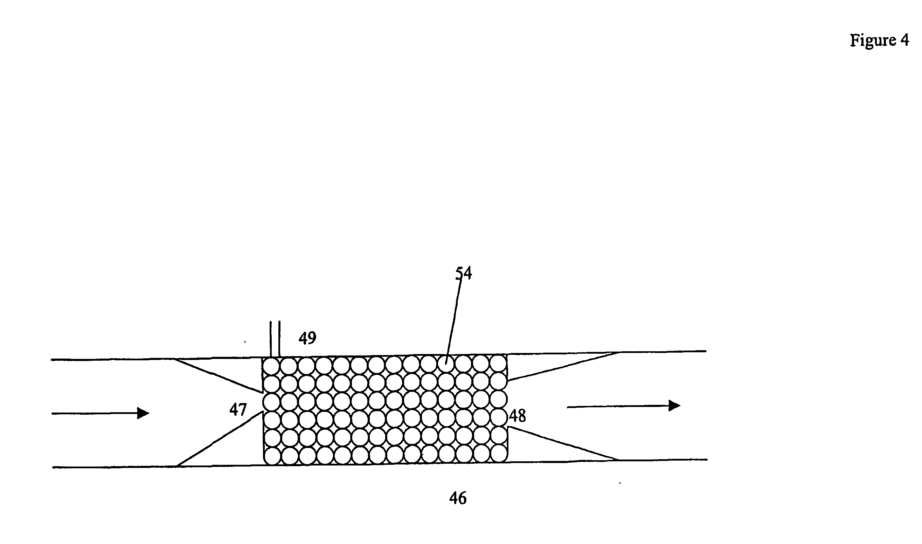

Fig. 4 schematically represents a further embodiment of mixing chamber 46 having a portion filled with beads 54.

It should be noted that in the case of an emergency situation, e.g. where rapid microbial decontamination is required, the system of the invention may by a skilled operator be converted into a disinfection and sterilisation unit.

The cleanness of the equipment is evaluated as a function of the ozone concentrations measured by ozone measuring means 5 and 16, respectively, i.e. the ozone concentration $[O_3]_5$ of the aqueous ozone solution in the mixing tank and the ozone concentration $[O_3]_{16}$ of the flow after passage through the equipment 14. The measured values, compensated for temperature and pressure variations, may be processed by the data processing unit 35, 36, to express cleanness of the equipment 14 as a ratio r of $[O_3]_{16}$ and $[O_3]_5$, which ratio theoretically may vary from 0 to 1:

$$r = [O_3]_{16}/[O_3]_5.$$

The calculated value of the ratio r will vary between 0 in a very dirty system and ideally 1 in a perfectly clean system. In practice, however, a ratio of 0.98-0.99 will in general not be exceeded due to e.g. the autodecomposition of ozone.

Ozone measurements being made in parallel by ozone measuring means 19, a ratio r of $[O_3]_{19}$ and $[O_3]_5$ may be calculated as a means of verification:

$$r' = [O_3]_{19}/[O_3]_5.$$

Theoretically this ratio should be equal to 1; however as stated above, due to auto-decomposition of ozone this ratio may be somewhat less than 1, e.g. 0.98-0.99.

Also, optionally a ratio r'', such as

$$r'' = [O_3]_{16}/[O_3]_{19}$$

may be calculated as representative of the cleanness of the equipment 14, taking account of the autodecomposition of ozone. In general, however, $r'' \cong r$.

The variation of the value of the ratio chosen for assessing the cleanness of the equipment over time may be visualized on the display screen of computer 36. A calculated value which substantially differs from 0, e.g. a value above 0.8, or above 0.9, say above 0.95, and which is essentially stable over e.g. 3-5 minutes may be taken as an indication that equipment 14 is essentially devoid of ozone consuming agents, i.e. essentially clean.

The data processing and control unit 35, 36 may be programmed to provide for an indication of the cleanness character of the equipment as a function of the calculated value of the ratio chosen for assessing the cleanness of the equipment. E.g. a score system of the type according to Table 3 may be adopted.

Table 3:

| Score system for assessing the cleanness character of an equipment. | |
|---|---|
| Value of ratio r for assessing the cleanness of the equipment | Cleanness character of equipment |
| $0 \leq r \leq 0.2$ | dirty |
| $0.2 < r \leq 0.7$ | moderately dirty |
| $0.7 < r \leq 0.9$ | moderately clean |
| $0.9 < r \leq 1$ | clean |

Of course, other limit values may be selected as a function of a preliminary calibration.

Also, programming may be made such that when r is inferior to a pre-selected threshold value, cleaning of the equipment is automatically initiated.

Results may be displayed on the screen of computer 36 and/or printed by means of printer 37.

Some examples of application of the invention will now be given.

EXAMPLE

Test plates were prepared as follows:

Known amounts of milk and bacteria were smeared onto the surface of SS 2343 stainless steel plates, 43x23 mm$^2$, in a number of 8, and then allowed to dry at room temperature for one hour.

The choice of bacteria and the procedure for drying of the treated stainless steel plates conformed to the European proposal for a standard test (prEN 13697) for the measurement of a disinfecting effect on microorganisms on surfaces.

The following microorganisms were used as test organisms: Pseudomonas *fragi* (ATCC 4973), Staphylococcus *aureus* (ATCC 6538), Enterococcus *hirae* (ATCC 10541), *Escherichia coli* (ATCC 10536) as well as spores from two different strains of *Bacillus cereus* (dairy strain 341 (isolated from butter) and type strain 229 (NCTC 2599)).

Clean SS 2343 stainless steel plates, 43x23 mm$^2$, in a number of 8, without any added microorganisms and/ or milk, were used as a control.

The degree of contamination of the test and control plates respectively was verified by two independent methods based on sampling and culturing the microorganisms on agar plates and counting the colonies in a microscope.

Based on the counts, the plates were divided into three groups: moderately contaminated and heavily contaminated test plates and the control plates comprising the non-contaminated group.

Assessment of the cleanness of the plates within each group then was performed by a method according to the invention applying a device according to the invention on a laboratory scale.

To this end, the plates were introduced into loops of silicone tubing. The loop containing the plate corresponds to equipment 14 in Fig.1, and a set-up essentially in accordance with the embodiment of Fig. 1 was used.

As fluid phase municipal water was used, having the following measured characteristics:

| pH | 7.9 |
|---|---|
| Temp | 17.7°C |
| Conductivity | 0.20 ms/cm |
| redox potential | 447 mV |

The water flow rate was about 5 litres/minute.

Ozone was measured simultaneously and at regular intervals over 5 minutes in two points, by use of two polarographic membrane electrodes, respectively corresponding to ozone measuring means 5 and 16 in Fig.1. Initial ozone concentrations were selected within a range of from 1 to 5 mg/l.

The results are presented in Figures 2-4. The bars filled with dashed lines represent the ozone reference concentration values measured in the reference point situated on ozone measuring loop in fig.1; the spotted bars represent the ozone concentration values measured in a point downstream of the silicone tubing loop containing the plate.

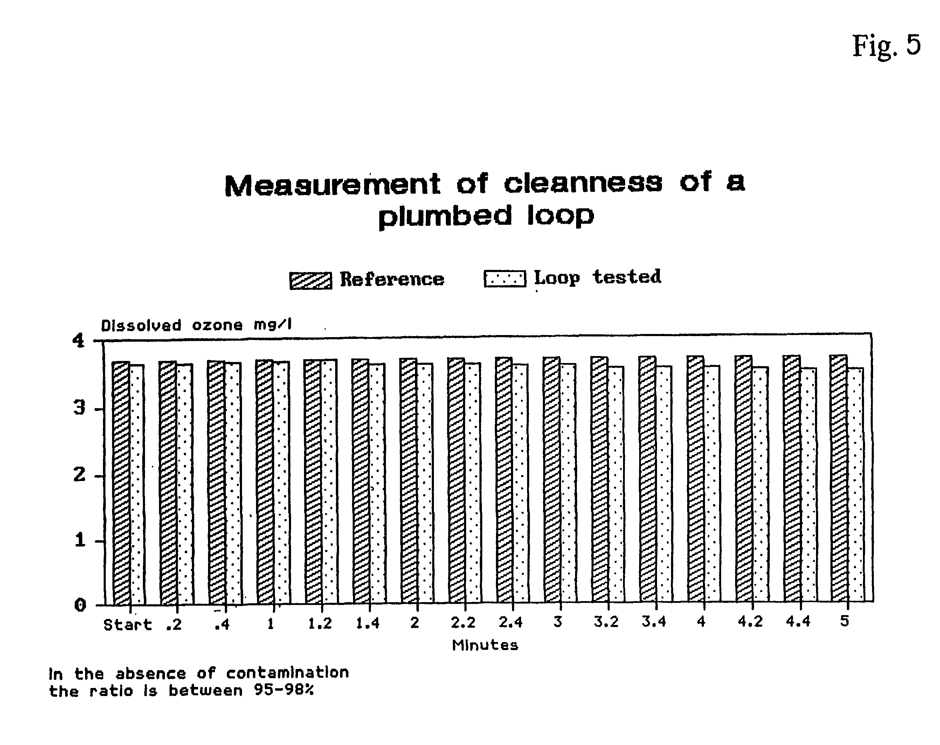

Fig. 5 represents the average results obtained in the control group. As can be seen, the initial ozone concentration measured in the reference point was approximately 3.7 mg/l whereas the initial concentration measured downstream of the loop was only slightly lower, around 3.6 mg/l, giving r=0.97 (according to the above formula for r). After a period of five minutes, the concentration measured in reference point was practically unchanged whereas the concentration measured downstream of the loop had decreased only slightly to a value around 3.5, giving an r value of 0.94. According to the score system given in Table 3 herein above, this value lies within the interval representative of a clean system.

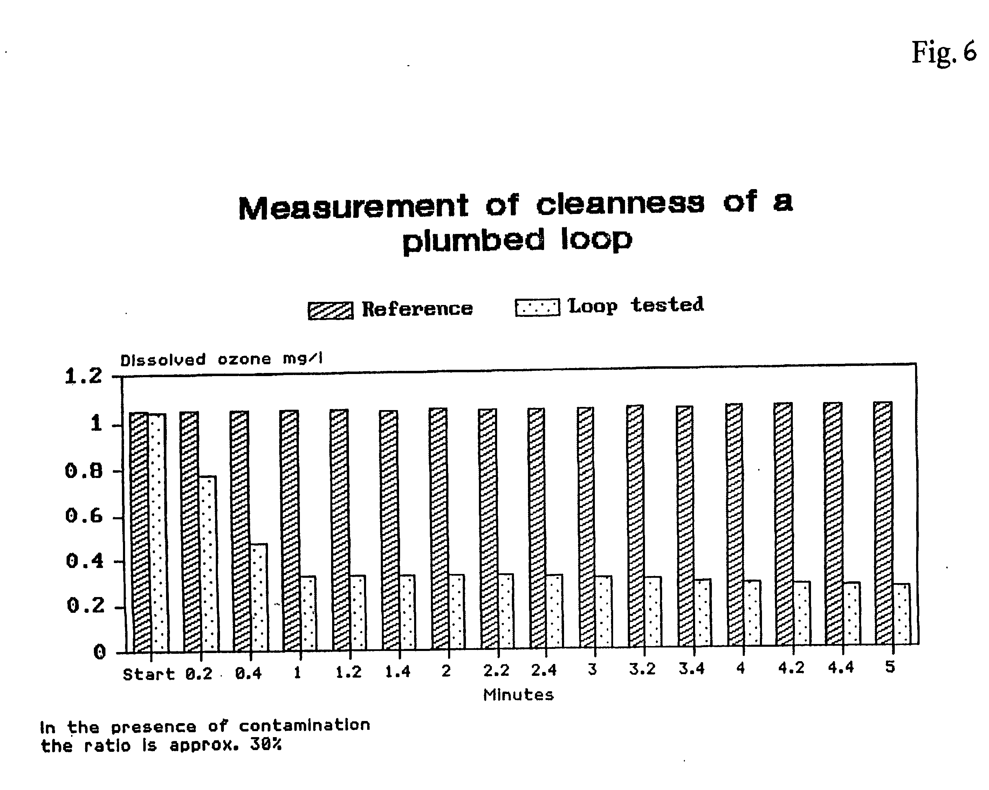

Fig. 6 represents the average results obtained in the moderately contaminated test group. As can be seen, the ozone concentration initially measured in the reference point was approximately 1.05 mg/l and remained virtually constant during the whole test period. The concentration measured downstream of the loop initially was only slightly lower than the one measured in the reference point. However, after only 0.2 minute it had decreased to 0.75 mg/l; after 0.4 minute, it had decreased to around 0.5 mg/l; after 1 minute it had reached a value of about 0.3 mg/l; and it then remained virtually constant during the rest of the test period. According to the above formula, this corresponds to a calculated value of r of approximately 0.3, representative of a moderately dirty system according to Table 3. As this value is obtained already within 1 minute from the start of the test, assessment of the cleanness consequently is very rapid.

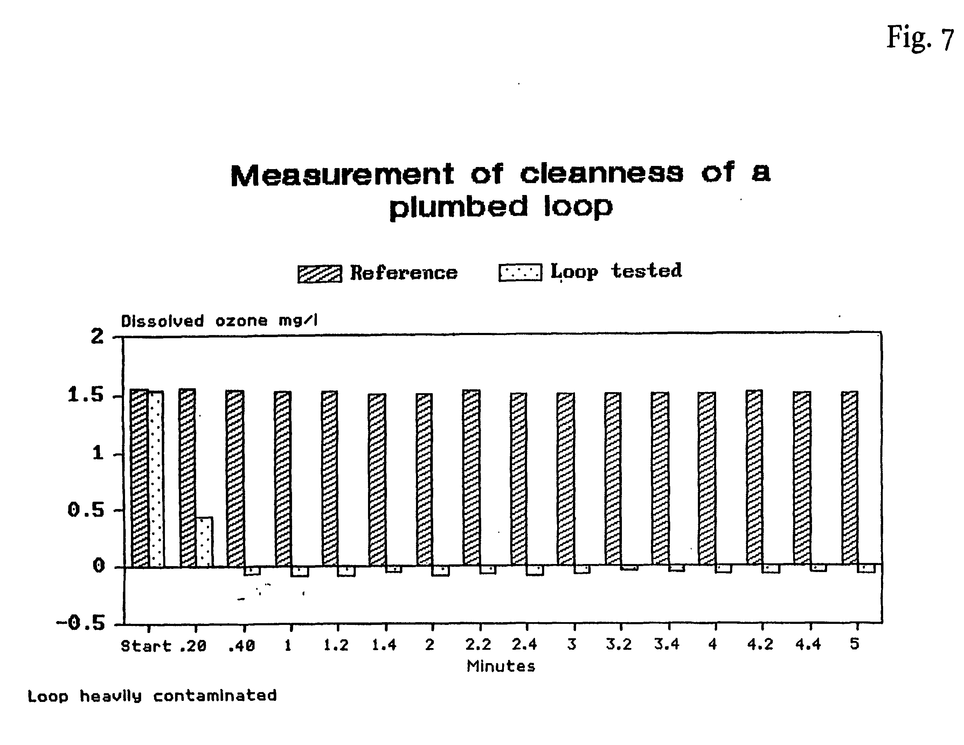

Fig. 7 represents the average results obtained in the heavily contaminated test group. Here, the initial ozone concentration measured in the reference point was approximately 1.6 mg/l and remained virtually constant during the whole test period (5 minutes). The initial concentration measured downstream of the loop initially was only slightly lower, but after only 0.2 minute it had decreased to less than 0.5 mg/l and after 0.4 minute, it had decreased to 0, and remained there for the rest of the test period. (The apparently negative value of the ozone concentration from 0.4 minute and onwards is an artefact inherent to the polarographic membrane electrode.) According to the Table 3, this corresponds to a dirty system. This value is obtained already at 0.4 minute from the start of the test, again showing the remarkable rapidity of the inventive method for assessing cleanness.

Whereas an embodiment of the invention has been described in detail herein above, it should be noted that the invention should not be construed to be limited thereto. On the contrary, many variations and modifications, which may come into the mind of the skilled reader, will fall within the scope of the present invention, as defined by the claims.
The method described may very advantageously be applied in a processing equipment, such as found within the food processing or pharmaceutical industry. The method could very conveniently be applied to a medical device such as catheter, where biofilms may be susceptible of forming.

**Claims**

1. A method of assessing the state of cleanness of an enclosed volume by

    (i) providing a fluid containing ozone at a concentration from 5 ppb to 10 ppm by weight;
    (ii) passing a continuous flow of the fluid through the enclosed volume;
    (iii) measuring intermittently or continuously the concentration of ozone in the fluid at a point located downstream of the enclosed volume ($[O_3]_{downstream}$);
    (iv) measuring intermittently or continuously the concentration of ozone in the flow of fluid containing ozone in at least one reference point where the fluid has not entered the enclosed volume ($[O_3]_{reference}$);
    (v) calculating the ratio r of $[O_3]_{downstream}$ to $[O_3]_{reference}$; and
    (vi) evaluating the state of cleanness of the enclosed volume as a function of the value of r, wherein at least one reference point, giving $[O_3]_{reference,bypass}$, is located in a flow line bypassing the enclosed volume.

2. A method according to claim 1, wherein at least one reference point is located upstream of the enclosed volume giving $[O_3]_{reference,upstream}$.

3. A method according to claim 2 wherein a ratio of $[O_3]_{reference,bypass}$ to $[O_3]_{reference,upstream}$ is calculated as a means of verification.

4. A method according to any of the claims 1-3 wherein a calibration of the r values as a function of the cleanness of the system is performed.

5. A method according to any of the claims 1-4, wherein cleaning of the enclosed volume is automatically initiated when the value of r is inferior to a pre-selected threshold value.

6. A method according to any of the claims 1-5, wherein the fluid is an aqueous liquid.

7. A method according to claim 6 wherein a value of r above 0.8, more preferably above 0.9, most preferably above 0.95, which is essentially stable over a time period of 3-5 minutes, is taken as an indication that the enclosed volume is essentially clean.

8. A method according to any of the claims 1-7 wherein the enclosed volume is part of a processing equipment for the food, beverage or pharmaceutical industry or part of a medical equipment, such as a catheter.

**Patentansprüche**

1. Verfahren für das Prüfen des Reinheitszustandes eines eingeschlossenen Volumens durch:

    (i) Bereitstellung eines Fluids, das Ozon in einer Konzentration von 5 ppb bis 10 ppm Gewichtsanteilen enthält;
    (ii) Durchleiten eines fortlaufenden Fluidstroms durch das eingeschlossene Volumen;
    (iii) periodisches oder fortlaufendes Messen der Ozonkonzentration in dem Fluid, an einem Punkt, der stromabwärts des eingeschlossenen Volumens liegt ($[O_3]_{stromabwärts}$);
    (iv) periodisches oder fortlaufendes Messen der Ozonkonzentration in dem Fluidstrom, der Ozon an mindestens einem Bezugspunkt enthält, an dem das Fluid nicht in das abgeschlossene Volumen eingedrungen ist ($[O_3]_{Bezug}$);
    (v) Berechnung der Kennzahl r von $[O_3]_{stromabwärts}$ bis $[O_3]_{Bezug}$; und
    (vi) Bewertung des Reinheitszustandes des eingeschlossenen Volumens als eine Funktion des Wertes von r, wobei mindestens ein $[O_3]_{Bezug, Umgehung}$ genannter Bezugspunkt sich in einer, das eingeschlossene Volumen umgehenden, Stromlinie befindet.

**2.** Verfahren nach Anspruch 1, worin mindestens ein $[O_3]_{Bezug,stromaufwärts}$ genannter Bezugspunkt sich stromaufwärts des eingeschlossenen Volumens befindet.

**3.** Verfahren nach Anspruch 2, worin eine Kennzahl von $[O_3]_{Bezug, Umgehung}$ bis $[O_3]_{Bezug,stromaufwärts}$ als ein Prüfungsmittel ermittelt wird.

**4.** Verfahren nach einem der Ansprüche 1- 3, worin eine Kalibrierung der r-Werte als eine Funktion der Reinheit des Systems durchgeführt wird.

**5.** Verfahren nach einem der Ansprüche 1- 4, worin eine Reinigung des eingeschlossenen Volumens automatisch eingeleitet wird, wenn der Werte r niedriger ist als ein zuvor gewählter Schwellenwert.

**6.** Verfahren nach einem der Ansprüche 1- 5, worin das Fluid eine wässrige Flüssigkeit ist.

**7.** Verfahren nach Anspruch 6, worin ein Wert r über 0,8, besser über 0,9 und noch besser über 0,95 liegt, der im Wesentlichen über eine Zeitspanne von 3 - 5 Minuten stabil ist, was als Anzeichen interpretiert wird, dass das eingeschlossene Volumen im Wesentlichen rein ist.

**8.** Verfahren nach einem der Ansprüche 1- 7, worin das eingeschlossene Volumen Teil einer Bearbeitungsanlage für die Nahrungsmittel-, Getränke- oder Pharmaindustrie oder Teil eines medizinischen Gerätes, wie z. B. einem Katheter, ist.

**Revendications**

**1.** Procédé d'évaluation de l'état de propreté d'un volume clos consistant à

(i) fournir un fluide contenant de l'ozone à une concentration de 5 ppb à 10 ppm en poids;
(ii) faire passer un flux continu de ce fluide à travers le volume clos;
(iii) mesurer, de façon intermittente ou continue, la concentration en ozone du fluide au niveau d'un point situé en aval du volume clos ($[O_3]_{aval}$);
(iv) mesurer, de façon intermittente ou continue, la concentration en ozone du flux de fluide contenant de l'ozone au niveau au moins d'un point de référence où le fluide n'a pas pénétré dans le volume clos ($[O_3]_{référence}$);
(v) calculer le rapport r de $[O_3]_{aval}$ sur $[O_3]_{référence}$; et
(vi) évaluer l'état de propreté du volume clos en fonction de la valeur de r,

dans lequel au moins un point de référence, donnant ($[O_3]_{référence, dérivation}$ se situe dans une ligne de courant d'écoulement évitant le volume clos.

**2.** Procédé selon la revendication 1, dans lequel au moins un point de référence est situé en amont du volume clos, donnant $[O_3]_{référence, amont}$.

**3.** Procédé selon la revendication 2, dans lequel un rapport de $[O_3]_{référence, dérivation}$ sur $[O_3]_{référence, amont}$ est calculé en tant que moyen de vérification.

**4.** Procédé selon l'une quelconque des revendications 1 à 3, dans lequel on réalise un étalonnage des valeurs r en fonction de la propreté du système.

**5.** Procédé selon l'une quelconque des revendications 1 à 4, dans lequel le nettoyage du volume clos est automatiquement amorcé lorsque la valeur de r est inférieure à une valeur seuil prédéterminée.

**6.** Procédé selon l'une quelconque des revendications 1 à 5, dans lequel le fluide est un liquide aqueux.

**7.** Procédé selon la revendication 6, dans lequel une valeur de r supérieure à 0,8, de manière plus particulièrement préférée supérieure à 0,9, de manière tout particulièrement préférée supérieure à 0,95, qui est sensiblement stable sur une durée de 3 à 5 minutes, est considérée comme une indication que le volume clos est essentiellement propre.

8. Procédé selon l'une quelconque des revendications 1 à 7, dans lequel le volume clos est une partie d'un équipement de traitement pour l'industrie alimentaire, l'industrie des boissons ou l'industrie pharmaceutique ou une partie d'un équipement médical, tel qu'un cathéter.

Fig. 1

Figure 2

EP 1 390 082 B1

49

47    50a    50b    48

46

Figure 3

Figure 4

Fig. 5

## Measurement of cleanness of a plumbed loop

▨ Reference   ⬚ Loop tested

In the absence of contamination
the ratio is between 95-98%

EP 1 390 082 B1

Fig. 6

# Measurement of cleanness of a plumbed loop

$\boxtimes$ Reference     $\boxed{\cdots}$ Loop tested

In the presence of contamination
the ratio is approx. 30%

EP 1 390 082 B1

Fig. 7

# Measurement of cleanness of a plumbed loop

[////] Reference     [:::::] Loop tested

Dissolved ozone mg/l

Minutes

Loop heavily contaminated

EP 1 390 082 B1